(19) Europäisches Patentamt
European Patent Office
Office européen des brevets

(11) Numéro de publication: **0 209 430 B1**

(12) **FASCICULE DE BREVET EUROPEEN**

(45) Date de publication de fascicule du brevet: **17.06.92**

(51) Int. Cl.[5]: **C12P 21/02**, C07K 5/00, C07F 9/38, C08F 8/40, A61K 31/66, A61K 37/02

(21) Numéro de dépôt: **86401371.9**

(22) Date de dépôt: **23.06.86**

# (54) L-di ou tripeptides possédant une activité biologique utilisable en médecine humaine et vétérinaire, procédé pour leur obtention et médicament les contenant.

(30) Priorité: **28.06.85 FR 8509931**

(43) Date de publication de la demande:
**21.01.87 Bulletin 87/04**

(45) Mention de la délivrance du brevet:
**17.06.92 Bulletin 92/25**

(84) Etats contractants désignés:
**AT BE CH DE FR GB IT LI LU NL SE**

(56) Documents cités:
**EP-A- 0 002 822**
**EP-A- 0 031 527**
**FR-A- 2 298 335**
**US-A- 4 086 136**

**ANTIMICROBIAL AGENTS AND CHEMOTHERAPY, vol. 15, no. 5, mai 1979, pages 677-683; F.R. ATHERTON et al.: "Phosphonopeptides as antibacterial agents: rationale, chemistry, and structure-activity relationships**

(73) Titulaire: **INSTITUT DE RECHERCHES CHIMIOUES ET BIOLOGIOUES APPLIOUEES (I.R.C.E.B.A.) Société à responsabilité limitée dite:**
**62, Grande-Rue**
**F-78490 Vicq(FR)**

(72) Inventeur: **Le Breton, Monique**
**10, Rue Henri Muret**
**F-78650 Beynes(FR)**
Inventeur: **Moriniere, Jean-Luc**
**43, Avenue Dr Arnold Netter**
**F-75012 Paris(FR)**
Inventeur: **Danree, Bernard**
**59 bis, Boulevard Devaux**
**F-78300 Poissy(FR)**
Inventeur: **Chasseray, Odile**
**40, Rue Jean Rey**
**F-78220 Viroflay(FR)**
Inventeur: **Rousseau, Claude**
**30, Grande Rue**
**F-78910 Orgerus(FR)**
Inventeur: **Lacolle, Jean-Yves**
**Résidence du Parc**
**F-78860 Saint Nom La Breteche(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

(74) Mandataire: **Combe, André et al**
**CABINET BEAU DE LOMENIE** 55, rue d'Amsterdam
**F-75008 Paris(FR)**

## Description

La présente invention concerne des L-di ou tripeptides à propriétés biologiques ; elle concerne également un procédé de préparation de L-di ou tripeptides possédant lesdites propriétés et enfin elle concerne les médicaments à activité anti-bactérienne contenant un di ou tripeptide de ce type.

On a déjà décrit, par exemple dans le brevet US 4016148 et dans le brevet français 79 16924, des polypeptides, en particulier des di et tripeptides qui possèdent des propriétés antibactériennes particulièrement intéressantes. Ces polypeptides antibactériens ont une configuration L.

La préparation de composés à configuration exclusivement L est difficile et onéreuse lorsque l'on utilise des méthodes de chimie classique. Il a été préconisé, pour certains polypeptides obtenus à partir d'acides aminés carboxyliques, de faire appel à une catalyse enzymatique, l'enzyme utilisée étant par exemple du type chymotrypsine.

Il a été trouvé et c'est là un objet de la présente invention que l'on pouvait avantageusement effectuer la synthèse de di ou tripeptides phosphoniques de configuration L avec des vitesses de réaction et de rendements importants en utilisant une catalyse enzymatique au moyen d'une protéinase à thiol choisie parmi la papaïne, la bromélaïne et la ficine.

La présente invention concerne donc un procédé de synthèse de di ou tripeptides phosphoniques de configuration L par réaction d'un $\alpha$-aminoacide ou d'un dipeptide carboxylique avec un acide $\alpha$-aminophosphonique ou d'un acide $\alpha$-aminocarboxylique avec un dipeptide phosphonique de configuration L, ledit procédé étant caractérisé en ce que :

- On met en contact dans un milieu solvant aqueux un réactif choisi parmi un acide $\alpha$-aminé ou un dipeptide carboxylique de configuration L formé par la condensation de deux acides $\alpha$-aminocarboxyliques L ou DL avec un réactif choisi parmi les acides $\alpha$-aminophosphoniques de configuration DL ou les dipeptides phosphoniques de configuration L formés par la condensation d'une molécule dudit acide $\alpha$-aminophosphonique avec un acide $\alpha$-aminocarboxylique, ledit contact étant réalisé en utilisant au moins deux moles de l'acide $\alpha$-aminé à fonction phosphonique de configuration L pour une mole de l'acide $\alpha$-aminocarboxylique ou d'un dipeptide de configuration L, en présence d'une enzyme choisie parmi la papaïne et la chymopapaïne et que l'on effectue la réaction en milieu acide, à une température comprise entre environ 10 et environ 70° C, de façon à obtenir une séparation de phase entre le milieu réactionnel et le polypeptide formé.

La chymopapaïne, qui se trouve être aujourd'hui le constituant principal de la papaïne industrielle, constitue l'enzyme de choix pour effectuer ladite réaction.

Par solvant aqueux, on indique un liquide contenant de l'eau et dans lequel les réactifs de départ sont solubles. Ce solvant peut être notamment et de préférence constitué par de l'eau pure mais il peut être également constitué d'un solvant miscible à l'eau dans lequel on a ajouté la quantité d'eau nécessaire pour permettre la réalisation de la catalyse enzymatique.

Comme réactif de départ, on peut utiliser l'un des produits de chacune des classes suivantes :

A). un aminoacide carboxylique qui est soit un produit de configuration L, soit un mélange de produits de configuration L et D ;

. un dipeptide constitué par la réunion de deux aminoacides carboxyliques, ledit dipeptide étant en configuration L.

B). un acide $\alpha$-aminophosphonique de formule

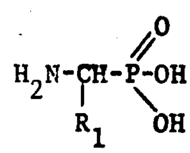

dans laquelle $R_1$ est H ou un radical alkyle (plus particulièrement le radical $CH_3$) ou aryle ; ledit acide pouvant être, lorsqu'il comporte un atome de carbone asymétrique, soit de forme L, soit constitué par un mélange de forme L et D.

. un dipeptide phosphonique constitué par la réunion d'un acide $\alpha$-aminophosphonique tel que défini ci-dessus avec un $\alpha$-aminoacide carboxylique, étant entendu que ledit dipeptide phosphonique est de configuration L.

Les aminoacides de la classe A sont utilisés avec blocage de leur fonction amine et les aminoacides de la classe B sont utilisés sous forme d'esters (notamment éthylique). La réaction réalisée dans l'invention consiste donc dans la formation d'une fonction amide par réaction de la fonction acide de l'aminoacide de la classe A avec la fonction amine de l'aminoacide de la classe B.

La concentration d'aminoacide de type B de structure L devra être, en moles, d'au moins deux fois celle de l'aminoacide de type A et de structure L.

La réaction est réalisée à une température comprise entre environ 10 et environ 70° C ; en fait la limite inférieure de la température correspond à une température à laquelle la réaction est trop lente notamment au stade industriel et la limite supérieure de la température correspond à la température de stabilité de l'enzyme utilisée.

Il est à noter que, contrairement à de nombreuses réactions enzymatiques, la réaction selon l'invention se déroule en milieu acide à un pH compris entre environ 4 et environ 5,5.

La quantité d'enzyme utilisée peut être variable il a été trouvé que la vitesse et le rendement final de la réaction augmentaient progressivement au fur et à mesure que la quantité d'enzyme utilisée augmente de 10 à environ 100 % de la quantité d'aminoacide carboxylique ou de dipeptide carboxylique (classe A) en configuration L utilisée comme produit de départ. Au-delà de cette quantité de 100 %, la vitesse et le rendement de la réaction varient peu.

Comme indiqué ci-dessus, la réaction doit être effectuée dans des conditions où le produit formé (di ou tripeptide) forme une phase distincte de celle constituée par le solvant aqueux de départ. Cette constitution de phase distincte peut se réaliser spontanément lorsque le produit formé précipite hors dudit milieu sous forme d'un solide ou d'une huile (cette dernière se déposant par exemple contre les parois du récipient dans lequel la réaction est effectuée) ; mais on peut également être amené à réaliser la réaction en présence d'un milieu biphasique constitué d'une part par le solvant aqueux mentionné et d'autre part par un liquide non miscible avec ledit solvant aqueux mais dans lequel le produit formé est très soluble.

Les exemples non limitatifs suivants illustrent l'invention en ce qui concerne le procédé de préparation de di ou tripeptides phosphoniques de configuration L.

## Exemple 1

Synthèse de l'acide L -Alanyl- L-(amino-1)-éthylphosphonique.

Cet exemple servant de référence, les opérations successives y seront décrites en détail.

### I. Synthèse et induction asymétrique de l'ester éthylique de l'acide (N-benzyloxycarbonyl)-L-Alanyl-L-(amino-1)-éthylphosphonique.

Dans un réacteur de 50 l équipé d'une agitation efficace, on introduit successivement :

- 2760 ml de soude normale (2,76 moles)
- 615 g de (N-benzyloxycarbonyl)-L-Alanine (2,76 moles)
- 2000 g d'ester éthylique de l'acide DL-(amino-1)-éthylphosphonique (11,05 moles)
- 32 g de chlorhydrate de L-cystéine
- 3,3 l de solution tampon citrique à pH : 4,5

Le milieu réactionnel limpide dont le pH est 6,92 est porté à 50 °C.

A cette température, on additionne une solution de 600 g de papaïne (50 nKat/mg) dans 0,8 l d'eau. Le pH évolue lentement et se stabilise à 6,84.

Par addition d'acide citrique en poudre (m = 1100 g), on amène le pH à 4,53 (pH compris entre 4,5 et 4,6). A ce stade, la solution est limpide.

On introduit alors, sous bonne agitation, 19 l de tétrachlorure de carbone.

L'avancement de la réaction et la cinétique sont suivis en HPLC en injectant, toutes les heures, 1 $\mu$l de solution organique sur une colonne O.D.S. Hypersil, 5$\mu$ (S.F.C.C) en éluant par un mélange (MeOH : 67; $H_2O$ : 30 ; $NH_4OH$ conc. : 3) - ($V_R$ = 7,2 - 7,4 ml).

Au bout de 15 h (temps de réaction compris entre 10 et 15 h), la synthèse est terminée; à ce stade, la concentration du produit est environ 0,14 molaire dans la phase organique.

On refroidit le milieu réactionnel, décante les deux phases et lave la phase organique à l'eau puis la concentre à sec après séchage sur sulfate de sodium :

m = 1250 g

Le résidu est repris par 14 l de chlorure de méthylène; après lavages successifs à l'acide chlorhydrique à 5% (4 l), à l'eau (4 l), à la soude 0,1 N (4 l) puis à l'eau (6 l) jusqu'à pH : 6,5, on concentre à sec sous vide.

On obtient 1020 g (2,64 moles) du produit cherché, soit un rendement de 96 % ; le produit obtenu présente, dans le méthanol, un pouvoir rotatoire de $[\alpha]_D^{20}$ = -30 ° ± 2 °, (c = 1).

### II. Synthèse de l'acide L-Alanyl-L-(amino-1)-éthylphosphonique optiquement pur :

L'huile obtenue est dissoute dans cinq volumes d'une solution d'acide bromhydrique à 33 % dans l'acide acétique, soit 5 l. La solution est laissée sous agitation pendant 5 h à la température ambiante puis versée sur 20 l d'éther isopropylique. Après cristallisation du bromhydrate de l'acide L-Alanyl-L-(amino-1)-éthylphosphonique sur les parois du récipient, on décante la phase éthérée et reprend le résidu par 5 l de méthanol.

A cette solution limpide, on ajoute 0,6 l d'oxyde de propylène dissous dans 1 l de méthanol. Un précipité apparaît rapidement et on le laisse cristalliser pendant 2 h à 0 ° C. On filtre, lave au méthanol et sèche à l'étuve sous vide à 60 ° C.

On obtient 502 g (2,56 moles) du produit, soit un rendement de 97 % ; ledit produit présente, dans l'eau, un pouvoir rotatoire de $[\alpha]_D^{20}$ = -22 ° ± 2 °, (c = 1).

Le solide obtenu est dissous dans 1,3 l d'eau bouillante et,à la solution décolorée par du noir animal 2 SA, on ajoute à 70 ° C, 3,5 l d'éthanol sous bonne agitation.

Un précipité cristallin apparaît au refroidissement à la température ambiante. Au bout de 2 h à 0 ° C, on filtre et sèche le produit à l'étuve sous vide à 60 ° C.

On obtient 351 g (1,79 moles) du produit cherché soit un rendement de 70 % ; ledit produit présente une température de fusion de 289-292 ° C et un pouvoir rotatoire dans l'eau de $[\alpha]_D^{20}$ = -45 ° ± 2 ° , (c = 1).

Le rendement global en L-Alanyl-L-(amino-1)-éthylphosphonique optiquement pur, exprimé par rapport au (N-benzyloxycarbonyl)-L-Alanine, est 65%.

III. Recyclage de l'ester éthylique de l'acide (amino-1)éthylphosphonique après épimérisation :

Pour réaliser le recyclage de l'ester éthylique de l'acide (amino-1)éthylphosphonique, on peut utiliser l'une ou l'autre des procédures suivantes :

A - Procédure par voie acide

La phase aqueuse du milieu réactionnel enzymatique, soit environ 9,5 l [qui contient 1520 g ( 8,41 mole) de l'ester éthylique de l'acide (amino-1)éthylphosphonique], est acidifiée par l'acide chlorhydrique concentré jusqu'à normalité finale de 6 N.

On porte au reflux cette solution pendant 5 h; après refroidissement, on décolore par passage sur noir 2 SA et élimine les impuretés minérales et organiques par percolation sur une colonne de résine S 861 (DUOLITE).

Les éluats acides sont concentrés à sec sous vide. Le résidu est dissous dans 2,7 l de méthanol auquel on ajoute, sous bonne agitation, de la pyridine sèche, jusqu'à pH 4,5.

Le précipité abondant qui apparaît est laissé à 0 ° C pendant 2 h.

On obtient ainsi 1020g (8,17 mole) d'acide DL-(amino-1)éthylphosphonique.

Le produit obtenu a un point de fusion supérieur à 260 ° C et un pouvoir rotatoire dans l'eau de $[\alpha]_D^{20}$ = 0 ° (c = 1).

On estérifie ce composé par les méthodes usuelles et, après distillation, on obtient 1207 g (6,67 mole) d'ester éthylique de l'acide DL-(amino-1)éthylphosphonique que l'on peut recycler.

B - Procédure par voie basique

La phase aqueuse du milieu réactionnel enzymatique, soit environ 9,5 l [qui contient 1520 g (8,41 moles) de l'ester éthylique de l'acide (amino-1)éthylphosphonique], est rendue basique jusqu'à pH 7,5 par addition d'environ 2 l de soude 4 N.

Cette solution est extraite par trois fois 8 l de dichlorométhane et les phases organiques jointes sont séchées sur sulfate de sodium et concentrées à sec.

m = 1420 g Rendement : 93%

L'huile jaune pâle obtenue est distillée sous pression réduite pour parfaire l'épimérisation de l'énantiomère D en excès.

m # 1400 g $Eb_{0,5\ mm\ Hg}$ = 70-72 ° C

Après trifluoroacétylation et séparation sur colonne chirale (L) SP 300 (5%, 1 m) [Inj. = Det = 250 ° C; isotherme à 140 ° C], on vérifie que l'ester récupéré contient 50% de l'isomère L ($t_R$ = 4,4 min) et 50% de l'isomère D ($t_R$ = 5,2 min). $[\alpha_D^{20}$ = 0 ° ± 0,5 ° , c = 100].

Le produit obtenu avec un rendement global voisin de 93% est ainsi recyclé selon I.

(L'épimérisation est également observée par stockage pendant 24 h de la solution organique d'extraction).

Dans l'état actuel des connaissances, il apparaît que le procédé par voie basique est préférable.

Exemple 2

Synthèse de l'acide (L)-phénylalanyl-aminométhylphosphonique.

Dans un erlenmeyer de 500 ml équipé d'un thermomètre et d'une agitation magnétique, on introduit :

- 33 ml de soude normale (0,033 mole),
- 10 g de (N-benzyloxycarbonyl)-DL-phénylalanine (0,033 mole),
- 5,51 g d'ester éthylique de l'acide aminométhylphosphonique (0,033 mole).

A cette solution limpide, on additionne 0,420 g de chlorhydrate de cystéine, 42,5 ml de tampon citrique à pH 4,5 et 60 ml d'eau.

La concentration des substrats est 0,22 molaire, le pH est compris entre 7,3 et 7,6.

Le mélange est porté à 40 ° C et on ajoute 5 g de papaïne (à 70 n Kat/mg) dissoute dans 45 ml d'eau. Le pH est amené à 5,5 par addition d'acide citrique en poudre (m = 10 g).

Le milieu réactionnel se trouble légèrement et une huile, qui cristallise lentement, se dépose sur les parois. On laisse la réaction se poursuivre pendant 8 h à 50 ° C.

L'analyse chromatographique montre que, dès le début, le dépôt est le dipeptide.

On refroidit, extrait par 3 x 50 ml de chloroforme et lave les phases organiques jointes à l'eau, à la soude 0,1 N puis à l'eau.

On sèche sur sulfate de sodium et concentre à sec. L'ester éthylique de l'acide (N-benzyloxycarbonyl)-L-phénylalanyl - aminométhylphosphonique cristallise.

On a obtenu 6,23 g (0,0139 mole) de l'ester, soit un rendement de 84 % ; le produit obtenu

présentait une température de fusion (banc kofler) de 102-104 ° C et un pouvoir rotatoire dans l'éthanol de $[\alpha]_D^{20}$ = -9,8 ° ± 2 °,(c = 1).

Le produit précédent est repris par 35 ml d'une solution à 33 % d'acide bromhydrique dans l'acide acétique ; le mélange réactionnel est laissé sous agitation pendant 5 h, puis versé sur 260 ml d'éther sulfurique. Après cristallisation du bromhydrate de l'acide L-phénylalanyl-aminométhylphosphonique sur les parois du récipient, on décante la phase éthérée et reprend le résidu par 30 ml de méthanol. On ajoute 4 mld'oxyde de propylène dissous dans 8 ml de méthanol. Un précipité cristallin se forme rapidement ; après 2 h à 0 ° C, on filtre et sèche à l'étuve sous vide à 60 ° C. On obtient ainsi 3,2 g d'acide L-phénylalanyl-aminométhylphosphonique optiquement pur.

L'acide obtenu (rendement 89,2 %) présente un point de fusion de 265-268 ° C et un pouvoir rotatoire dans l'eau de $[\alpha]_D^{20}$ = +74,8 ° ± 2 ° ,(c = 1).

De la même façon, on peut récupérer et recycler, à partir de la phase aqueuse enzymatique, l'excès de l'ester éthylique de l'acide aminométhylphosphonique (cf. exemple 1).

Exemple 3

Synthèse de l'acide (L) Leucyl-aminométhylphosphonique.

Dans un erlenmeyer de 500 ml muni d'un thermomètre et d'une agitation magnétique, on introduit :

- 20 ml de soude normale (0,020 mole),
- 5,3 g de (N-benzyloxycarbonyl)-L-Leucine (0,020 mole),
- 6,2 g de l'ester éthylique de l'acide aminométhylphosphonique (0,037 mole),

A la solution limpide, on ajoute 240 mg de chlorhydrate de cystéamine dissous dans 5,7 ml d'eau, puis 24 ml de tampon citrique à pH : 4,5 et 100 ml d'eau. La solution est à pH : 7,53.

On chauffe à 40 ° C par l'intermédiaire d'un bain-marie thermostaté et ajoute 1,71 g de papaïne (à 26 n Kat/mg) dissous dans 21 ml d'eau ; le pH évolue à 7,25.

Par addition d'acide citrique (m = 8 g), le pH est amené à 5,6. Très rapidement une huile décante sur les parois du récipient et cristallise lentement.

Au bout de 8 h, on refroidit et extrait le précipité formé par 120 ml de chlorure de méthylène. Après lavages successifs par 30 ml d'eau, 30 ml d'acide chlorhydrique à 5 %, 30 ml d'eau, 30 ml de soude 0,1 N et 30 ml d'eau (pH : 6,5) la phase organique est séchée sur sulfate de sodium et concentrée à sec.

On obtient ainsi 6,9 g de l'ester éthylique de l'acide L-Leucyl-aminométhylphosphonique. Cet ester a un point de fusion (banc kofler) de 82-83 ° C et un pouvoir rotatoire dans le méthanol de $[\alpha]_D^{20}$ = - 25 ° ± 2 °,(c = 1).

Ce produit est repris par 33 ml de solution d'acide bromhydrique à 33 % dans l'acide acétique et laissé sous agitation pendant 6 h. On ajoute 250 ml d'éther sulfurique et le résidu décanté est dissous dans 30 ml de méthanol. On ajoute 6 ml de méthanol et 3,5 ml d'oxyde de propylène.

Un précipité apparaît qu'on laisse cristalliser pendant 2 h à 0 ° C. On filtre et sèche sous vide à 60 ° C.

On obtient ainsi 3,54 g d'acide L-Leucyl-aminométhylphosphonique.

L'acide obtenu a une température de fusion de 243-247 ° C et un pouvoir rotatoire dans l'eau de $[\alpha]_D^{20}$ = +60,5 ° ± 2 °, (c = 1).

Dans le cas de l'utilisation d'un solvant organique chloré, le temps de réaction est de 6 h et le rendement est voisin de 85 %.

Exemple 4

Synthèse de l'acide L-Alanyl-L-Alanyl-L-(amino-1)-éthylphosphonique.

I. Synthèse de l'acide (N-Benzyloxycarbonyl)-L-Alanyl-L-Alanine :

a) Synthèse de l'ester éthylique de l'acide (N-Benzyloxycarbonyl)-L-Alanine.

Dans un erlenmeyer de 500 ml équipé d'une agitation magnétique, d'un thermomètre et d'un réfrigérant à eau, on introduit :

- 20 g de (N-Benzyloxycarbonyl)-DL-Alanine (0,0897 mole),
- 45 ml de soude 2 N,
- 0,27 g de chlorhydrate de L-cystéine,
- 27 ml de solution tampon citrique à pH : 4,5.

La solution est portée à 50 ° C ; à cette température le pH est 5,6. On ajoute 10 g de papaïne (à 70 n Kat/mg) ; au bout de quelques minutes le pH évolue vers 5,3.

Par addition d'acide citrique en poudre (m : 1,5 g), on amène le pH à 4,53.

Au milieu réactionnel, on ajoute une solution de 10,3 g d'éthanol (0,223 mole) dans 330 ml de chloroforme.

La réaction d'estérification est suivie en HPLC (colonne O.D.S Hypersil, 5$\mu$ (S.F.C.C.) ; solvant : $MeOH/H_2O/NH_4OH$ conc. = 67/30/3 ; $V_R$ = 5,3 - 5,4 ml). Au bout de 3 h, la réaction n'évolue plus.

On refroidit et décante les phases ; la phase aqueuse est extraite par 2 x 100 ml de chloroforme ; les phases chloroformiques jointes sont lavées par 100 ml d'eau, 100 ml de bicarbonate de sodium à 5 % puis 2 x 100 ml d'eau jusqu'à pH = 6,5.

Après séchage sur sulfate de sodium, on concentre à sec.

On obtient ainsi 0,028 mole de l'ester éthylique de l'acide (N-Benzyloxycarbonyl)-L-Alanine.

L'ester obtenu est une huile dont le pouvoir rotatoire, dans le méthanol, est : $[\alpha]_D^{20}$ = -31,9° ± 2°,(c = 1).

b) Synthèse de l'ester éthylique de l'acide (N-Benzyloxycarbonyl)-L-Alanyl-L-Alanine.

Après déprotection du produit précédent par une solution d'acide bromhydrique à 37 % dans l'acide acétique [d'après J.R. COGGINS, N.L. BENOITON Can. J. Chem. (1971) 49 1968], on obtient 3,16 g d'ester éthylique de la L-Alanine (0,027 mole, Rdt : 90 %).

Dans un erlenmeyer de 250 ml, on introduit :

- 9,62 g de (N-Benzyloxycarbonyl)-DL-Alanine (0,043 mole),
- 21,5 ml de soude 2 N,
- 3,16 g d'ester éthylique de la L-Alanine (0,027 mole),
- 0,26 g de chlorhydrate de cystéamine,
- 26 ml de solution tampon citrique à pH = 4,5.

On chauffe la solution à 50°C et,à cette température, on ajoute 4,8 g de papaïne (à 70 n Kat/mg) ; le pH évolue lentement vers 5,9.

Par addition d'acide citrique en poudre, on ajuste le pH à 4,52, puis on introduit, sous bonne agitation, 120 ml de chloroforme.

La réaction suivie en HPLC, comme dans les exemples précédents, est complète en 6 h.

La phase organique, traitée comme dans l'exemple 4-I-a donne 6,23 g de l'ester éthylique de l'acide (N-benzyloxycarbonyl)-L-Alanyl-L-Alanine.

m = 6,23 g (0,01935 mole) huile

c) Synthèse de l'acide (N-Benzyloxycarbonyl)-L-Alanyl-L-Alanine.

Le composé précédent traité par une solution méthanolique de soude 2 N [selon J.R. COGGINS, N.L. BENOITON Can. J. Chem. (1971) 49 1968] donne 5,4 g de (N-Benzyloxycarbonyl)-L-Alanyl-L-Alanine.

On a obtenu 5,4 g (0,0184 mole) d'un produit dont le point de fusion est de 152-154°C et le pouvoir rotatoire dans le méthanol de $[\alpha]_D^{20}$ = -35° ± 2°, (c = 0,5).

II. Synthèse de l'ester éthylique de l'acide (N-Benzyloxycarbonyl)-L-Alanyl-L-Alanyl-L-(amino-1)-éthylphosphonique.

Dans un erlenmeyer de 100 ml, on introduit successivement :

- 5,29 g de (N-Benzyloxycarbonyl)-L-Alanyl-L-Alanine (0,018 mole),
- 9 ml de soude 2N,
- 13 g de l'ester éthylique de l'acide DL-(amino-1)-éthylphosphonique (0,072 mole),
- 0,28 g de chlorhydrate de L-cystéine,
- 28 ml de tampon citrique à pH : 4,5,
- 5 g de papaïne (à 70 n Kat/mg).

Selon le procédé décrit dans l'exemple 1-I et, après 10 h de réaction, on obtient l'ester éthylique de l'acide (N-Benzyloxycarbonyl)-L-Alanyl-L-Alanyl-L-(amino-1)-éthylphosphonique [F° : 127-129°C ; $[\alpha]_D^{20}$ = - 53° ± 2° (1,méthanol)] que l'on déprotège selon le procédé décrit en 1-II pour obtenir après recristallisation 2,73 g d'acide L-Alanyl-L-Alanyl-L-(amino-1)-éthylphosphonique optiquement pur.

Le produit obtenu a un point de fusion de 277-279°C et un pouvoir rotatoire dans l'eau de $[\alpha]_D^{20}$ = -68,5° ± 2° (c = 1).

L'excès de substrat phosphoné est récupéré et recyclé selon l'exemple 1-III à partir de la phase aqueuse du milieu réactionnel enzymatique (m = 7,24 g,Rdt : 74 %).

Exemple 5

Synthèse du sel de l'amino-2-(hydroxyméthyl)-2-propanediol-1,3 avec l'acide L-Alanyl-L-(amino-1)-éthylphosphonique.

Dans un erlenmeyer de 100 ml, on introduit successivement :

- 15 ml d'eau distillée,
- 5 g (25,5 mmoles) d'acide L-Alanyl-L-(amino-1)éthylphosphonique,
- 3,1 g (25,6 mmoles) d'amino-2-(hydroxyméthyl)-2 propanediol-1,3.

La solution homogène du sel est maintenue sous agitation pendant 2 h à 40°C.

Après concentration à sec à l'évaporateur rotatif, le résidu est repris par 20 ml d'éthanol 100% et concentré à nouveau à sec.

Le résidu visqueux est cristallisé par agitation dans 50 ml d'éthanol 100%, refroidi à 0-5°C.

Le produit cristallisé obtenu est filtré, lavé sur filtre à l'éthanol 100% et séché au dessicateur sous vide en présence d'agent déshydratant (silicagel).

m = 8 g Rendement : 99%

Ce composé présente un point de fusion de 255-260°C/décomposition et un pouvoir rotatoire dans l'eau de :

$[\alpha]_D^{20}$ = - 17,9° ± 2,6°,(c = 1).

Exemple 6

Synthèse du produit d'addition d'une résine sulfonique polystyrénique avec l'acide L-Alanyl-L-(amino-1)éthylphosphonique.

Sur une colonne de verre (L = 30 cm, diamètre = 12,5 cm) qui contient 100 ml de résine (conditionnée en cycle acide), on percole à la vitesse de 5 ml/min une solution aqueuse de l'acide L-Alanyl-L-(amino-1)éthylphosphonique. On vérifie, par un test à la ninhydrine, l'absence de fuite du composé phosphoné.

Après lavages à l'eau, dès la saturation, on essore la résine chargée du principe actif, et la sèche à l'étuve sous vide à 40 ° C.

m = 83 g % Acide (P) : 19,5%

Le dosage du composé fixé sur la résine est réalisé par une percolation, sur une colonne de chromatographie, d'une solution d'ammoniaque à 5% et une colorimétrie des éluats après addition de ninhydrine.

Exemple 7

En opérant comme ci-dessus, on a pu obtenir notamment les produits suivants :

a) En condensant l'acide (N-Benzyloxycarbonyl)-L-Leucyl-L-Leucine avec l'ester éthylique de l'acide DL-(amino-1)-éthylphosphonique, on obtient l'acide L-Leucyl-L-Leucyl-L-(amino-1)-éthylphosphonique avec un rendement global de 64,5%.

Le produit obtenu présente un point de fusion de 265-268 ° C et un pouvoir rotatoire dans la soude 0,1 N de $[\alpha]_D^{20}$ = - 35 ° ± 2 °, (c = 1).

b) L'acide Sarcosyl-L-Alanyl-L-(amino-1)-éthylphosphonique qui a un point de fusion de 243-247 ° C et un pouvoir rotatoire dans l'eau de $[\alpha]_D^{20}$ = - 83,5 ° ± 2 °, (c = 1).

c) L'acide Glycyl-L-Tyrosyl-aminométhylphosphonique ayant un point de fusion d'environ 300 ° C (avec décomposition) et un pouvoir rotatoire dans l'eau de $[a]_D^{20}$ = + 15 ° ± 2 ° ,(c = 1).

d) L'acide L-(nitro-3)-Tyrosyl-aminométhylphosphonique ayant un point de fusion de 255-257 ° C et un pouvoir rotatoire dans la soude 0,1 N de $[\alpha]_D^{20}$ = + 9,7 ° ± 1 °, (c = 0,25).

e) L'acide Glycyl-L-Valyl-aminométhylphosphonique ayant un point de fusion de 278-281 ° C et un pouvoir rotatoire dans l'eau de $[\alpha]_D^{20}$ = - 35 ° ± 2 ° ,(c = 0,5).

f) Le sel de la L-Arginine avec l'acide L-Alanyl-L-(amino-1)éthylphosphonique ayant un point de fusion de 200-202 ° C et un pouvoir rotatoire dans l'eau de $[\alpha]_D^{20}$ = - 9,6 ° ± 2 ° , (c = 1).

g) L'acide Sarcosyl-L-Leucyl-L-(amino-1)-éthylphosphonique ayant un point de fusion de 270-274 ° C et un pouvoir rotatoire dans l'eau de $[\alpha]_D^{20}$ = - 58 ° ± 2,5 °, (c = 1).

h) L'acide L-Alanyl-Sarcosyl-aminométhylphosphonique ayant un point de fusion de 252-255 ° C et un pouvoir rotatoire dans l'eau de $[\alpha]_D^{20}$ = + 17,4 ° ± 2,5 ° ,(c = 1).

i) le produit d'addition d'une résine fortement basique polystyrénique avec l'acide L-Alanyl-L-(amino-1)éthylphosphonique, qui contient 12 % de l'acide cité.

j) le produit d'addition d'une résine polyamine phénolique faiblement basique avec l'acide L-Alanyl-L-(amino-1)éthylphosphonique, qui contient 10 % de l'acide cité.

## Revendications

**1.** Procédé de synthèse de di ou tripeptides phosphoniques de configuration L par réaction d'un α-aminoacide ou d'un dipeptide carboxylique avec un acide α-aminophosphonique ou acide α-aminocarboxylique avec un dipeptide phosphonique de configuration L, ledit procédé étant caractérisé en ce que :

- on met en contact dans un milieu solvant aqueux un réactif choisi parmi un acide α-aminé ou un dipeptide carboxylique de configuration L formé par la condensation de deux acides α-aminocarboxyliques L ou DL avec un réactif choisi parmi les acides α-aminophosphoniques de configuration DL ou les dipeptides phosphoniques de configuration L formés par la condensation d'une molécule dudit acide α-aminophosphonique avec un acide α-aminocarboxylique, ledit contact étant réalisé en utilisant au moins 2 moles de l'acide aminé à fonction phosphonique de configuration L pour 1 mole de l'acide aminocarboxylique ou de dipeptide de configuration L, en présence d'une enzyme choisie parmi la papaïne et la chymopapaïne, et que l'on effectue la réaction en milieu acide, à une température comprise entre environ 10 et environ 70 ° C, et de façon à obtenir une séparation de phase entre le milieu réactionnel et le polypeptide formé.

**2.** Procédé selon la revendication 1, caractérisé en ce que l'enzyme utilisée est la chymopapaïne ou toute papaïne industrielle composée essentiellement de chymopapaïne.

**3.** Procédé selon l'une des revendications 1 et 2, caractérisé en ce que la séparation de phase entre le milieu réactionnel et le polypeptide formé est réalisée par utilisation d'un liquide non miscible audit milieu mais dans lequel le produit obtenu est soluble.

## Claims

1. Process for synthesis of phosphonic di or tripeptides of L-configuration by reaction of an alpha-aminoacid or a carboxylic dipeptide with an alpha-aminophosphonic acid or an alpha-aminocarboxylic acid with a phosphonic dipeptide of L-configuration, said process being characterized in that:
   - a reagent selected from an alpha-amino acid or a carboxylic dipeptide of L-configuration, formed by the condensation of two L or DL alpha-aminocarboxylic acids, is placed in contact in an aqueous solvent medium with a reagent selected from the alpha-aminophosphonic acids of DL configuration or the phosphonic dipeptides of L-configuration formed by the condensation of a molecule of said alpha-aminophosphonic acid with an alpha-aminocarboxylic acid, said contact being made by using at least two moles of the amino acid with phosphonic function of L-configuration for one mole of the aminocarboxylic acid or dipeptide of L-configuration, in the presence of an enzyme selected from papain and chymopapain, and in that the reaction is carried out in an acid medium, at a temperature of between about 10 and about 70 °C, so as to obtain a separation of phase between the reaction medium and the polypeptide formed.

2. Process according to claim 1, characterized in that the enzyme used is chymopapain or any industrial papain essentially composed of chymopapain.

3. Process according to one of claims 1 and 2, characterized in that the phase separation between the reaction medium and the polypeptide formed is effected by using a liquid nonmiscible with said medium but in which the product obtained is soluble.

## Patentansprüche

1. Verfahren zur Synthese von phosphonischen Di- oder Tripeptiden der L-Konfiguration durch Umsetzung einer α-Aminosäure oder eines Carboxyldipeptids mit einer α-Aminophosphonsäure oder einer α-Aminocarbonsäure mit einem phosphonischen Dipeptid der L-Konfiguration, welches Verfahren dadurch gekennzeichnet ist, daß:
   - in einem wässerigen Lösungsmittelmilieu ein Reagens, ausgewählt aus einer α-Aminosäure oder einem Carboxyldipeptid der L-Konfiguration, gebildet durch die Kondensation zweier L- oder DL-α-Aminocarbonsäuren, mit einem Reagens, ausgewählt aus α-Aminophosphonsäuren der DL-Konfiguration oder phosphonischen Dipeptiden der L-Konfiguration, gebildet durch die Kondensation eines Moleküls dieser α-Aminophosphonsäure mit einer α-Aminocarbonsäure, in Kontakt gebracht wird, welcher Kontakt unter Verwendung von mindestens 2 Mol der Aminosäure mit Phosphonfunktion der L-Konfiguration pro 1 Mol der Aminocarbonsäure oder des Dipeptids der L-Konfiguration in Gegenwart eines Enzyms, ausgewählt aus Papain und Chymopapain, stattfindet, und daß die Reaktion in saurem Milieu bei einer Temperatur zwischen etwa 10 und etwa 70 °C und derart durchgeführt wird, daß eine Phasentrennung zwischen dem Reaktionsmilieu und dem gebildeten Polypeptid erzielt wird.

2. Verfahren nach Anspruch 1, dadurch gekennzeichnet, daß das verwendete Enzym Chymopapain oder jedes industrielle Papain ist, das im wesentlichen aus Chymopapain besteht.

3. Verfahren nach einem der Ansprüche 1 und 2, dadurch gekennzeichnet, daß die Phasentrennung zwischen dem Reaktionsmilieu und dem gebildeten Polypeptid durch Verwendung einer mit diesem Milieu nicht mischbaren Flüssigkeit, in der aber das erhaltene Produkt löslich ist, erfolgt.